# EUROPEAN PATENT APPLICATION

(11) **EP 2 596 975 A1**
(43) Date of publication of application: **29.05.2013**
(21) Application number: 11811876.9
(22) Date of filing: 19.07.2011
(51) Int. Cl.: B60K 28/06, B60R 25/04, G01N 33/497, G06K 9/00

(54) **IDENTIFICATION AND SECURITY DEVICE USING SHORT-DISTANCE OCULAR BIOMETRY**

(30) Priority: 20.07.2010 ES 201031111
(71) Applicant: Valls Chaparro, Isaac, 08221 Terrassa (Barcelona) (ES); López Roca, Sergio, 08221 Terrassa (Barcelona) (ES)
(72) Inventor: CALDERÓN OLIVERAS, Alberto, E-08221 Terrassa (Barcelona) (ES)
(74) Representative: Maldonado Jordan, Julia
(86) International application number: PCT/ES2011/070533
(87) International publication number: WO 2012/013849

(57) **Abstract**

The invention relates to a device for preventing identity thefts, in order to avoid the starting of mechanical apparatus, such as a vehicle (automobile or similar), or the switching-on of electrical, electronic or electro-medical equipment, if the user is not the authorised user or if the physical conditions necessary to guarantee the safety of the user or other people are not met. The device comprises: a first module comprising a mouthpiece for the acquisition of data such as blood alcohol concentrations or exhalation pressure, a second module provided with a central processing unit, and a third module for capturing images of the eye of the user, provided with a microcamera and means for illuminating the eye. The preferred distance between the eye of the user and the microcamera is between 2 cm and 5 cm.

## Description

### Object of the invention

The invention refers to an identification and security device using short-distance ocular biometry.

### Field of the invention

The field of the invention is mainly the prevention of eventual supplantations of the identity of persons in different situations, such as access control, payment operations, operation of machines, medical applications and similar. As a particular case, it comprises as well the prevention of traffic accidents derived by the driving of vehicles by persons with an excess of alcohol or drugs in blood, which excess may be revealed by means of an alcohol test or a test which is specific for drugs, obtained by means of the person involved blowing into an apparatus designed for said finality.

The above mention of vehicles has to be extended to all means permitting the movement of persons or goods from one place to another, as well as all types of heavy machinery, agricultural machinery and special machinery, provided with a thermal or electric engine.

### Background of the invention

Means are already known nowadays for the obtainment of results similar to those above mentioned, for the enhancement of safety for the drivers of vehicles. However, all of those means are based in principles and embodiments different from the present invention.

A formerly known means consists in an alcohol meter capable to immobilize vehicles in case of detecting an excess of alcohol in the breath of an eventual driver. In said apparatus, which has been designed by the present applicant, the optical exploration of the individual is carried out from a relatively long distance (for instance, in the order of 10 to 15 centimetres) between the eye and the apparatus, requiring the previous introduction of photographies of the iris of the eyes of the eventual drivers of the vehicle. The operation of blowing into the alcohol sensor has to be carried out at the same time than the inspection of the iris of the eyes of the driver pretending to start the vehicle.

US patent 6.748.792 and its corresponding European patent EP 1.591.296 refer to a system for the detection of the alcohol rate in the breath of an individual and its interpretation by a test apparatus associated with the starting system of the vehicle, without optical recognition of the identity of the user.

US patent 5.845.733 describes an antitheft device for vehicles which has means for the investigation of the retina of the presumed user without examination of the breath of this latter.

US patent 6.433.863 describes the combination of a breath analyser and sensor of the aspect of the eye of the user, which sensor operates at a given distance from the analyser, both elements being interrelated by a given set of circuits with doubtful reliability for the pretended objective.

Other prior documents having similar objectives to those of the present invention, based in different principles as to its concept, development and material embodiment, are those contained in the following patents: US 5.729.619, US 5.845.733, US 6.198.996, US 6,433.863, US 6.748.792, US 7.256.700, US 2002/0089660, US 2004/0239510, EP 0.955.219, EP 1.591.296, WO 02/22.407, WO 2006/116187, GB 2.415.036.

### Description of the invention

The device which is the object of the present invention is aimed at preventing the start of a mechanical operation such as starting a vehicle (an automobile or similar) or the starting of an electrical, electronic or electro-medical equipment in case that the driver is not authorised for said starting operation or that the user does not have the physical characteristics which are necessary to guaranty the safety of the driver himself or that of the user or, in its case, of the people accompanying him as well as the integrity of the vehicle or equipment.

To this aim, the device has a system for the recognition of the identity of the user, which in the particular case of driving a vehicle is associated to the introduction of a sample of the breath of the user at the moment of the investigation and a system associated to those two above permitting or preventing the start of the engine or equipment, depending on the level of alcohol or drugs contained in said sample.

A distance under five centimetres will be considered a short distance, this order of magnitude deserving to be remarked in comparison to other person recognition systems by means of the iris.

### Description of the figures

To facilitate the description, a block diagram and perspective views will be annexed, in which the concept and structure of an identification and security device by means of ocular biometry at a short distance have been represented, according to the principles of the claims.
- Figure 1 shows a diagram representing three main modules of the structure of the device of the invention, that is, a first module (1) for the acquisition of images and an illumination system, a second module (2) for measuring the alcohol or drugs rate and a third data processing module (3); additionally having elements for the interaction and communication between the user and the elements forming the device.
- Figure 2 is an outside perspective view of a practical embodiment of the device of the invention.
- Figure 3 is a perspective view, with a partial cross section, of the same device.

### Preferred embodiment of the invention

The elements designated with signs in the diagram and drawings correspond to the parts which are indicated in the following:
In the first module (1) aimed at the acquisition of data concerning the related parameters for the application of the device such as pressure and concentration of determined substances in the body of the user; the mouthpiece (11) is an element which permits the inflow of the stream of air inspirated by the user towards the pressure sensor (12) which senses the changes in pressure at the entry of the air and permits the activation of the device, and towards the sensor (13) which determines the concentration of alcohol or drugs in the inspirated stream of air, analysing the same.

The second module (2) associated to the first module (1) for the measurement of the concerned parameters includes a central processing unit (21). The keypad (22) permits the interaction with the device and the forced activation of the same using security codes. The central processing unit (21) is a microprocessor controlling the operation of the whole device or equipment.

The device contains additionally light indicators (24) which are assemblies of electroluminescent elements, such as LED, informing the user on the state of the device and the result of the tests.

The acoustical indicators (24) consist essentially in buzzers which guide the user during the test process.

In the third module (3), the microcamera (31) captures the images (form and dimensions) of the eye of the user, and the lighting unit (32) is the assembly of LED elements illuminating in a uniform and diffused way the eye cavity.

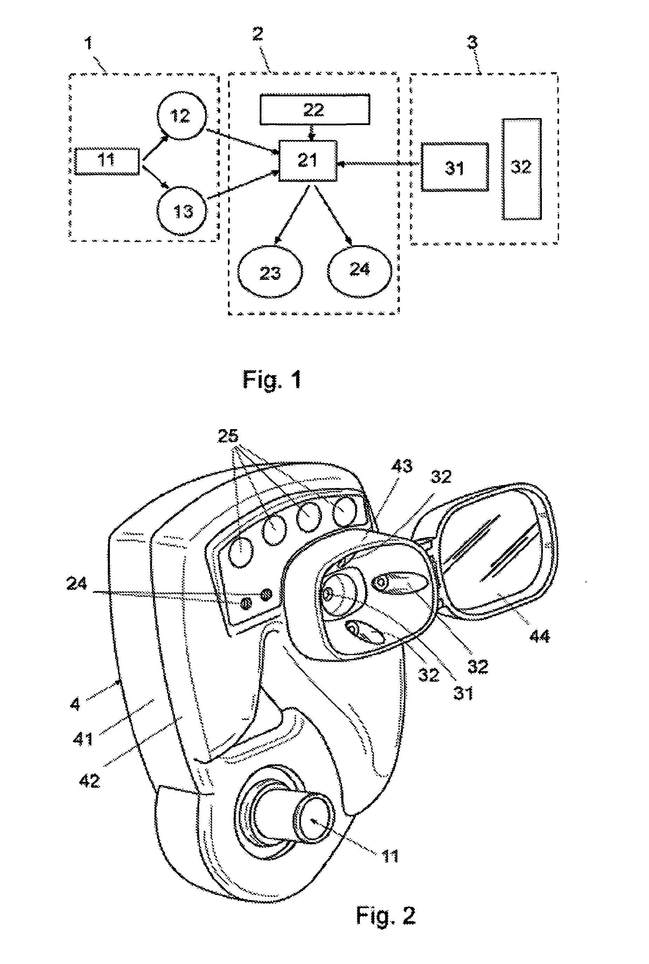

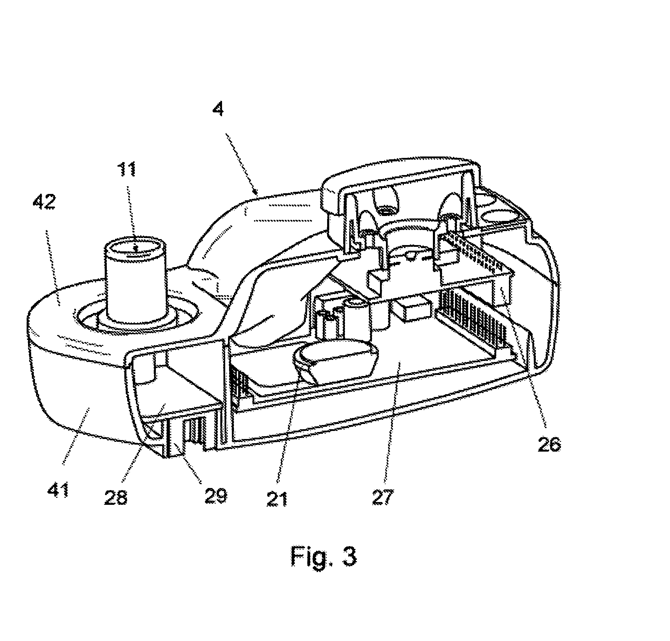

In figures 2 and 3, the references correspond to the following elements: (41 and 42) base and cover of the body (4), (11) the mouthpiece, (43) the visor, (31) the microcamera, (44) the cover of the visor, (24) the lighting indicators of the results, (25) the buttons for fast release in emergency cases, (26, 27, 28) the printed circuits with the different sections of the circuit, (29) an incorporated power source, (21) the central processing unit, (32) the LED elements of the illumination unit.

The described device constitutes an equipment combining the recording data provided by an alcohol or drugs meter with the biometric data of the user (its identification by means of the iris of the eyes), thus generating an outgoing signal.

The signal will be considered negative if:
- the user having access to the identification system is not registered, that is, is not recognised by the device;
- the user having access to the identification system is registered, but shows a rate of alcohol or drugs which exceeds the permitted level.

With the above mentioned elements, the operation of the device is as follows.

The user will place the device such that his right eye is directly looking to the microcamera (31) and the mouthpiece (11) is located between the lips. The right eye is located to a distance comprised between 3 and 5 cm, approximately, from the microcamera.

The device constantly senses and signals, operating as a monitor, the pressure variations by means of the pressure sensor (12). Whenever an important increase of the pressure level is sensed, an acoustical signal is activated by the indicator (24) and the calculation is started of the stream of air circulating through the group of sensors (12 and 13). When the stream of air exceeds a predetermined threshold, an image is taken from the eye of the user and the level of alcohol or drugs is stored.

The central processing unit (21) processes the image of the eye of the user converting the same in an unique code which will be compared to the previously stored codes in the memory means of the device. If the obtained code does not coincide with any of the stored codes, the result of the test is negative. In the contrary case, the user is identified. If the level of alcohol or drugs is lower than a maximum established threshold the result of the test is positive (favourable to the user); in the contrary case, the result of the test is negative (unfavourable to the user).

The results of all tests carried out are stored in the memory means of the device and afterwards they may be unloaded from a computer having the appropriate software means.

The keypad (25) permits to force a positive result by means of a secret code unique for each user. The activation by means of a secret code is foreseen for exceptional cases in which it is necessary and justified to have such positive result which, in its case will also be recorded in the memory of the device.

There is the possibility that the tests with the device have to be carried out periodically after starting the controlled apparatus and, in its case, the vehicle. The device would indicate to the user the moment in which the test should be repeated and it would record in the memory the result obtained or the fact of having omitted a periodical test.

In certain circumstances and according to the configuration of the device, the result may be simply informative (that is, the equipment recognizes the user and for example, the alcohol rate in blood, but it permits to starting the vehicle, in spite of having a level of alcohol in blood exceeding the maximum established threshold).

It has been foreseen that the central processing unit (21) be connected to a global positioning system such as GPS in which case it would be possible to identify the location of a vehicle incorporating the device of the invention in case of its intended use, for example, in the vicinity of a bar lounge or similar.

In a preferred embodiment, the GPS would be located within the body of the device making it more integrated and the GPS would permit to control theft intents or the non permitted use of the vehicle.

The device of the invention will incorporate a system (by means of cable or wireless) to communicate with other electronic pieces of equipment.

A preferred embodiment of the illumination system (32) will be based in the use of low consumption electroluminescent elements (LED) with reduced dimensions, operating in the 640 nm range and permitting to correctly illuminate the eye cavity for the preferable optical and camera characteristics. However, according to the applications foreseen for the device, the type of the illumination may be changed as well as the lighting elements and the frequency of operation.

The device will be able to operate with different systems of power supply such as the electrical battery of a vehicle, incorporated batteries or public mains.

After having sufficiently described the nature of the invention, as well as a preferred example of the embodiment, it is to be stated to all effects that the materials form, shape and arrangement of the elements which have been described will be subject to modifications whenever this does not mean an alteration of the essential characteristics of the invention which are claimed in the following.

## Claims

1. Identification and security device using short-distance ocular biometry, for the prevention of eventual supplantations of the identity of persons, particularly to prevent carrying out certain actions, **characterised by** comprising:
- a first module (1) for the acquisition of data concerning to the parameters involved in the application of the device, such as pressure and concentration of determined substances in the body of the user;
- a second module (2) associated to the first module (1) including a central processing unit (21) for the evaluation of the concerned parameters and illumination and acoustical indicating elements (23, 24) and,
- a third module (3) for the capture of images of the eye of the user provided with an illumination system (32).

2. Device, according to claim 1, **characterised in that** the first module (1) comprises a mouthpiece (11) for the inflow of the breath of the user, a pressure sensor (12) and a sensor (13) for the investigated parameter, such as the alcohol rate in blood.

3. Device, according to claim 1, **characterised in that** the second module (2) comprises the central processing unit (21) complemented with a keypad (22) for the interaction of the device and its activation by means of security codes, as well as acoustical indicators (24) and illumination indicators (23).

4. Device, according to claim 1, **characterised in that** the third module (3) includes a microcamera (31) which has its signal output coupled to a second module (2) and means for the local illumination (32), such as electroluminescent elements (LED).

5. Device, according to claim 1, **characterised in that** its practical embodiment is carried out by means of a portable hand operated apparatus formed by a body (4) having a base portion (41) and a functional cover (42) having the mouthpiece (11) for the inspiration, a visor (43) with a passage for the microcamera (31) and a protection cover (44), as well as illumination indicators (24), indicators of the operation of the device and push buttons (25) for fast release in cases of emergency.

6. Device, according to the previous claims, **characterised in that** it comprises printed circuit boards ( 26, 27, 28) corresponding to the operation of the microcamera, the central processing unit and the sensors, respectively, a power source (19) and the central processing unit (21), as well as electroluminescent elements (32) for the illumination of the eye cavity of the user and ports for connection with the electrical circuits of a vehicle and with a computer.

7. Device, according to the previous claims, **characterised in that** the preferred vision distance between the eye of the user and the microcamera (31) is comprised between 2 and 5 cm.
